# FASCICULE DE BREVET EUROPEEN

(11) **EP 3 644 909 B1**
(45) Date de publication et mention de la délivrance du brevet: **07.04.2021**
(21) Numéro de dépôt: 18749453.9
(22) Date de dépôt: 25.06.2018
(51) Int. Cl.: A61F 2/95

(54) **POIGNEE POUR DELIVRANCE D'UN STENT ET DISPOSITIF COMPRENANT LA POIGNEE**
GRIFF ZUR FREISETZUNG EINES STENTS UND VORRICHTUNG MIT DEM GRIFF
HANDLE FOR DELIVERING A STENT AND DEVICE COMPRISING THE HANDLE

(30) Priorité: 27.06.2017 FR 1755883
(43) Date de publication de la demande: 06.05.2020
(73) Titulaire: Hexacath, 92500 Rueil-Malmaison (FR)
(72) Inventeur: ASCHER, Gilles, Charles, Franklin, 92200 Neuilly-sur-Seine (FR); ANDREANI, Philippe, 75015 Paris (FR)
(74) Mandataire: Cabinet Beau de Loménie
(86) Numéro de dépôt international: PCT/FR2018/051539
(87) Numéro de publication internationale: WO 2019/002739

(56) Documents cités:
- WO-A1-2015/014960
- DE-A1-102006 004 123
- KR-A- 20110 023 243
- US-A1- 2007 168 014
- US-A1- 2010 004 606

## Description

L'invention concerne une poignée pour la délivrance d'un stent et un dispositif de délivrance d'un stent comprenant la poignée. L'invention concerne aussi une méthode de délivrance du Stent avec ladite poignée.

### ETAT DE LA TECHNIQUE

Il est connu par le document EP 1834610 un dispositif de délivrance d'un stent comprenant une poignée 212 qui comprend un boîtier 214 et une poulie 222, une tige de poussée 204 ayant une extrémité proximale 204P connectée au boîtier et une extrémité distale au voisinage de laquelle est localisé le stent et dans lequel la poignée a une longueur linéaire Y plus faible que la longueur linéaire X du stent. Il est prévu une gaine 206 de constriction du stent à sa partie distale et ayant une extrémité proximale 206P connectée à une extrémité distale d'un fil de rétractation 224 connecté à son extrémité proximale à ladite poulie 222. Ainsi, la rotation de la poulie 222 enroule le fil de rétractation qui rétracte la gaine 206 de constriction du stent en libérant ainsi le stent.

Cependant, l'inventeur a découvert que ce dispositif présente l'inconvénient d'exiger d'exercer une force de rétractation significative sur la poulie pour procéder au retrait de la gaine de constriction du stent.

Le document **D1** = US 2007/0168014 décrit une poignée de délivrance d'un stent comprenant un système de traction de la gaine de protection et constriction du stent avec des cordes 180 de traction, voir figures 15A, 15B,15C, dont une extrémité avant est fixée à un élément mobile dit SLIDER 152 qui est commun à tous les modes de réalisation, car c'est lui qui est relié à la gaine de protection du stent et sert à la traction de la gaine lors de son déplacement.

L'invention prévoit une poignée de conception simple comme décrit ci-après.

### BUTS DE L'INVENTION

L'invention a pour but principal de trouver une solution qui résolve le problème technique de réduire la force de traction qu'il faut exercer sur la poulie pour aboutir au retrait de la gaine et libérer le stent à l'extrémité distale.

L'invention a encore pour but principal de trouver une solution qui résolve le problème technique de réduire la force de traction qu'il faut exercer sur la poulie selon une solution qui démultiplie cette force de traction.

L'invention a encore pour autre but principal de trouver une solution qui résolve le problème technique de réduire la force de traction qu'il faut exercer sur la poulie, qui soit simple de mise en œuvre, sans augmenter le coût de fabrication et qui soit sûre et fiable techniquement et médicalement, permettant une fabrication à l'échelle industrielle.

### RESUME DE L'INVENTION

L'invention fournit une poignée pour délivrance d'un stent disposé sur un cathéter par mouvement de retrait d'un élément tubulaire ou gaine dit intermédiaire, de protection du stent, ayant une extrémité distale et une extrémité proximale, le stent est disposé de manière contrainte à l'intérieur de l'extrémité distale de l'élément tubulaire ou gaine dit intermédiaire comprenant un système de palan servant à la traction de l'élément tubulaire ou gaine dit intermédiaire de protection du stent sur une distance de retrait prédéterminée afin de découvrir le stent et permettre sa délivrance et sa dilatation, caractérisée en ce que le système de palan comprend en amont une boucle solidaire de l'élément tubulaire ou gaine dit intermédiaire de protection dans laquelle boucle passe un fil ayant une fonction de palan dont une extrémité est fixée au boîtier de la poignée pour être fixe et l'autre extrémité est solidaire d'une roulette de manœuvre montée à rotation sur le boîtier de la poignée.

Selon une autre variante de réalisation particulière, la poignée présente une partie avant en direction du stent et une partie arrière servant à l'introduction du cathéter sur lequel est disposé le stent, en position initiale, le système de palan se trouve vers l'avant de la poignée de manière que lors du retrait, par traction, la distance de retrait soit d'une longueur sensiblement proche de la longueur de la poignée.

Selon une autre variante de réalisation particulière de l'invention, la roulette précitée comprend un palier ou arbre latéral sur lequel vient s'enrouler le fil de traction.

Selon une autre variante de réalisation particulière de l'invention, la roulette avec son palier ou arbre latéral se trouve dans une position intermédiaire entre l'avant et l'arrière de la poignée, la partie arrière de la poignée étant d'une dimension permettant d'être tenue confortablement dans la main d'un opérateur. Dans cette position intermédiaire, l'opérateur peut tenir fermement la poignée tout en pouvant manœuvrer la roulette qui est d'une dimension suffisante pour avoir son bord circonférentiel externe faisant saillie à l'extérieur de la poignée.

Selon une autre variante de réalisation particulière, le bord extérieur circonférentiel de la poignée est cranté afin de faciliter l'opération de rotation exercée par un doigt de l'opérateur qui peut être le pouce lorsque la roulette est disposée en haut de la poignée.

Selon une autre variante de réalisation particulière de l'invention, il est prévu à l'intérieur de la poignée à l'arrière, un axe support de renvoi du fil de traction provenant de la partie avant de la poignée en passant dans la boucle, ce qui participe également à la diminution de la force de traction qu'il faut exercer sur la gaine pour la tracter à l'intérieur de la poignée. Ainsi, le fil de traction est fixé à un première extrémité à une embase solidaire du boîtier vers la partie arrière de la poignée, puis se dirige à l'avant de la poignée pour passer dans la boucle solidaire de la gaine de protection du stent, revient à l'arrière de la poignée pour passer autour de l'axe de support et de renvoi, et repart vers l'avant jusqu'à avoir sa deuxième extrémité fixée à la roulette. Ainsi, selon ce mode de réalisation, la longueur totale du fil de traction est supérieure à deux fois la longueur de la poignée et inférieure à trois fois la longueur de la poignée.

L'axe de support de renvoi disposé à l'arrière de la poignée peut être réalisé en un matériau facilitant le glissement, tel qu'en métal ou en matière plastique.

Selon une autre variante, la poignée présente à l'avant un élément de renfort servant à sa rigidification et à la connexion avec le dispositif cathéter comprenant le stent.

Selon une autre variante de réalisation particulière de l'invention, il est prévu un élément de blocage provisoire d'un déplacement du système de palan. Cet élément de blocage peut être prévu au niveau de la roulette en empêchant sa rotation et en particulier prévu au niveau de la boucle solidaire de l'élément tubulaire ou gaine intermédiaire, en étant introduit sous la boucle, par exemple sous forme d'une patte de section cylindrique ou tronconique, afin de soulever la boucle, et l'écraser pour la bloquer en position jusqu'au moment de l'opération de la poignée.

Selon un deuxième aspect, l'invention couvre aussi un système de délivrance d'un stent qui comprend la poignée précédemment décrite.

Selon une variante de réalisation particulière de l'invention, ce système de délivrance du stent comprend la poignée précitée et un dispositif cathéter, conformé pour être introduit dans un canal du corps humain, en particulier un vaisseau sanguin, une veine, une artère et notamment une coronaire, et comprenant un élément tubulaire ou gaine dit intermédiaire ayant une extrémité proximale et une extrémité distale ; un stent disposé de manière contrainte à l'intérieur de l'extrémité distale de l'élément tubulaire intermédiaire; un élément tubulaire ou gaine dit interne, ayant une extrémité proximale et une extrémité distale, disposé à l'intérieur de l'élément tubulaire dit intermédiaire et ayant une longueur suffisante pour que son extrémité distale vienne se positionner à l'arrière du stent et le bloquer en position, pour empêcher son retrait ; l'extrémité proximale de l'élément tubulaire interne est solidarisée à la poignée pour avoir une position fixe.

Selon une autre variante de réalisation particulière de l'invention, il est prévu un élément tubulaire ou gaine dit externe, ayant une extrémité proximale et une extrémité distale, qui est positionné à l'extérieur de l'élément tubulaire intermédiaire, d'une dimension plus courte que l'élément tubulaire intermédiaire pour avoir son extrémité distale disposée à une distance prédéterminée en amont du stent, l'extrémité proximale de l'élément tubulaire externe est solidarisée à la poignée pour avoir aussi une position fixe.

Selon une autre variante de réalisation de l'invention, l'extrémité distale de l'élément tubulaire intermédiaire présente un diamètre D1 plus grand sur toute la longueur où il reçoit le stent pour faciliter son insertion, puis présente une pente de jonction pour aboutir à un diamètre D2 plus petit sur la partie amont de l'élément tubulaire intermédiaire. Ce diamètre D1 est prévu pour correspondre sensiblement au diamètre D3 de l'élément tubulaire externe de manière à venir se positionner contre l'extrémité distale de l'élément tubulaire externe qui est fixe et qui constitue une butée d'arrêt du retrait de l'élément tubulaire intermédiaire.

L'homme de l'art comprend que l'élément tubulaire intermédiaire est prévu pour coulisser longitudinalement entre l'élément tubulaire interne et l'élément tubulaire externe qui restent de position fixe relativement à la poignée ; le stent restant en place car la position de l'extrémité distale de l'élément tubulaire interne reste fixe et empêche tout mouvement de retrait du stent. L'invention permet donc de délivrer le stent à une position précise.

Selon une variante de réalisation, la distance prédéterminée en amont du stent de l'extrémité distale de l'élément tubulaire ou gaine dit externe peut correspondre sensiblement à la distance de retrait de l'élément tubulaire intermédiaire, cette distance de retrait correspondant au moins à la longueur du stent à délivrer.

D'autres buts, caractéristiques et avantages de l'invention apparaîtront clairement à la lumière de la description explicative qui va suivre faite en référence aux figures annexées qui représentent un mode de réalisation actuellement préféré de l'invention donné simplement à titre d'illustration et qui ne saurait en aucune façon limiter la portée de l'invention. Les dessins font partie intégrante de l'invention et complètent ainsi la description.

Dans les dessins :
- la figure 1 représente une vue générale du système 1 de délivrance d'un stent 2 comprenant un dispositif cathéter 100 comportant à la partie distale la présence du stent 2, protégé à l'intérieur d'un élément tubulaire ou gaine de protection 120, avant sa libération, et une poignée 10 ;
- la figure 2 représente, de manière très agrandie, une vue en coupe de la partie distale du dispositif cathéter permettant de bien voir le stent 2, et les éléments tubulaires ou gaines respectivement interne 110, intermédiaire 120 logeant le stent 2, et externe 130 ;

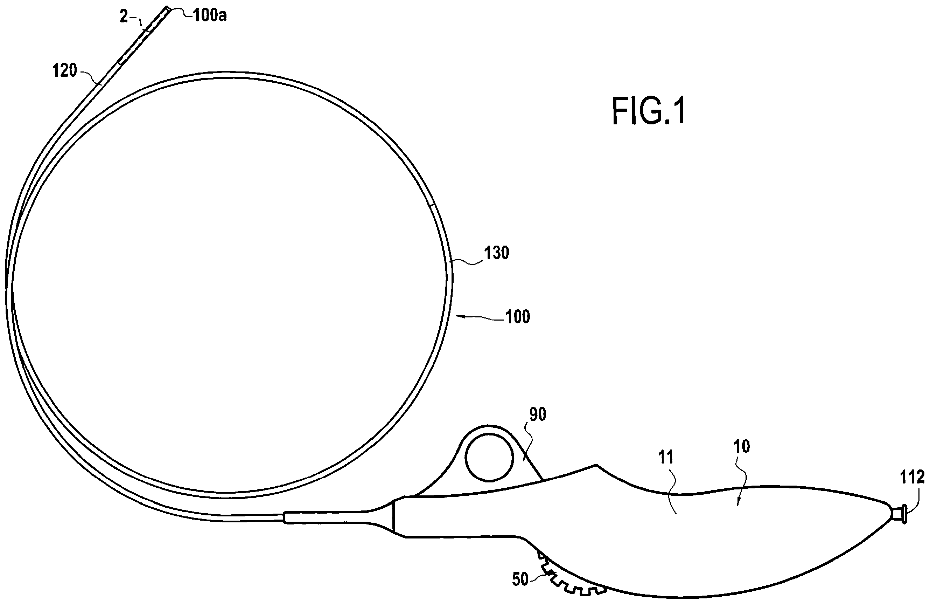

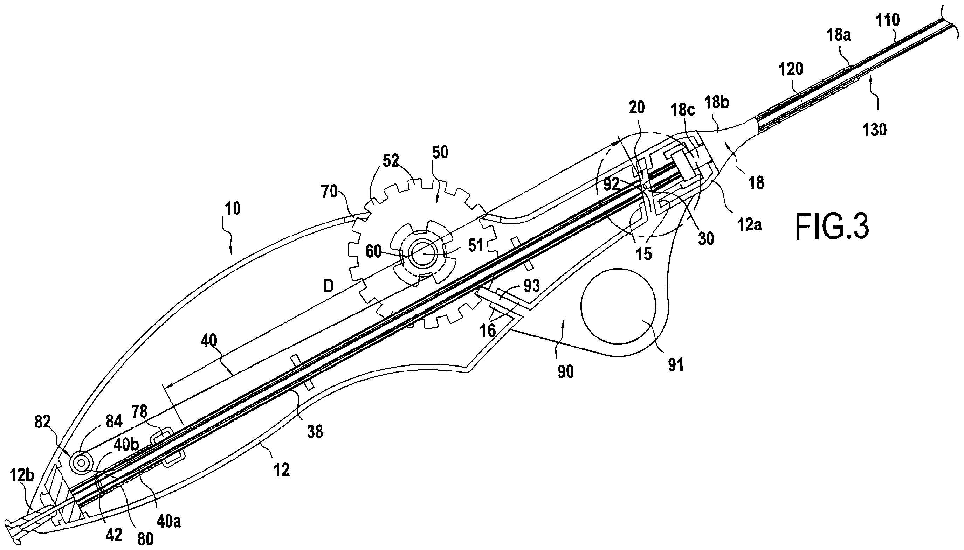

- La figure 3 représente une vue générale de la poignée, en position ouverte avec la coque droite visible à la figure 1 enlevée, la poignée étant réalisée en deux parties ou moitiés longitudinales complémentaires ou coques s'encliquetant l'une dans l'autre. La figure 3 montre ainsi la coque gauche 12 pour visualiser l'intérieur de la poignée qui comprend le système de palan selon l'invention avec sa roulette. La figure 3 montre aussi l'élément de renfort 18 en place ayant une partie avant tronconique disposée à l'extérieur de la poignée et une partie arrière en forme de T inversé dont la base du T vient se loger dans un logement transversal où il est maintenu en position fermée de la poignée. La figure 3 montre encore l'organe de blocage de palan 20, tel qu'une goupille et comprenant un orifice 91 pour faciliter son tirage et se terminant par deux doigts 92 et 93, l'un à l'avant 92 passant ici sous la boucle 30 et l'autre 93 venant s'insérer entre deux dents 52 de la roulette 50 ;
- la figure 4 représente de manière très agrandie la partie avant de la poignée avec le renfort enlevé pour avoir plus de visibilité et centrée sur le système de palan 20 ; on voit que le système de palan comprend une boucle et un fil de traction, et les deux jambes de la boucle sont bien collées à l'élément tubulaire intermédiaire ou gaine de logement et protection du stent ;
- la figure 5 représente une vue plus agrandie de la figure 3 de l'organe de blocage du palan 20 ;
- la figure 6 représente également de manière très agrandie la partie arrière de la poignée montrant l'embase 80 de fixation de la partie haute du fil 40 de traction sur le boîtier ou ici la coque 12 de la poignée et également l'axe support de renvoi du fil de traction en direction de la roulette ; cet axe support de renvoi étant important dans le cadre du système de palan selon l'invention afin de réduire la force de traction ;
- la figure 7 représente de manière agrandie la roulette 50 de traction permettant de voir l'arbre latéral 60 sur lequel s'enroule le fil 40 de traction ; et
- la figure 8 représente une vue générale de la poignée, en position ouverte, similaire à la figure 3 mais avec la boucle disposée en fin de traction à l'arrière de la poignée qui correspond à la position de libération du stent, comme cela est bien visible sur le zoom de la figure 8.

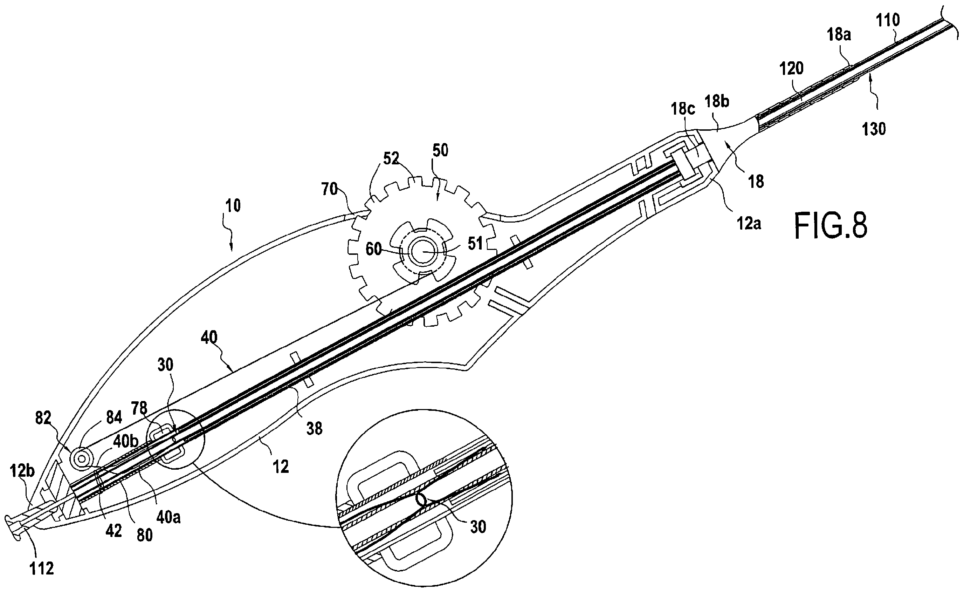

Selon un premier aspect, et en référence aux figures 1 à 8, l'invention concerne un nouveau concept de poignée (10) pour délivrance d'un stent (2) par mouvement de retrait d'un élément tubulaire ou gaine (120) de protection du stent (2), cet élément ou gaine peut être intermédiaire selon le mode de réalisation actuellement préféré et fait partie d'un cathéter (100) qui sera décrit plus loin. Il est aussi prévu un élément tubulaire (110) de blocage ou d'arrêt du stent (2), dit « push tubing » ou « stop tubing » et avantageusement un élément tubulaire externe ou gaine externe (130) facilitant l'introduction dans un canal tel qu'un vaisseau sanguin. Dans la suite de la description, on utilisera indifféremment les termes d'« élément tubulaire » ou de « gaine ».

Ce mode de réalisation actuellement préféré concerne aussi un système de délivrance d'un stent (2) qui comprend cette poignée (10) qui constitue l'essence de l'invention.

Le système de délivrance d'un stent comprend généralement un dispositif cathéter (100) conçu pour permettre une distance de retrait prédéterminé D définie en partie par la longueur de la poignée (10), afin de découvrir le stent (2) et permettre sa délivrance et sa dilatation. On comprend aisément que la distance de retrait prédéterminée D est aussi fonction de la longueur du stent (2) à libérer. Les longueurs de stent (2) à libérer sont variables et peuvent atteindre 150mm ou même 200mm. On comprend aisément que l'on peut prévoir plusieurs dimensions de poignées pour s'adapter à la longueur du stent (2).

Selon l'invention, la poignée est caractérisée en ce qu'elle comprend un système de palan (20) servant à la traction de la gaine intermédiaire de protection (120) du stent (2) sur une distance de retrait (D) prédéterminée afin de découvrir le stent et permettre sa délivrance et sa dilatation.

Selon une variante de réalisation particulière, la poignée est caractérisée en ce que le système de palan (20) comprend une boucle (30) solidaire de la gaine intermédiaire de protection (120) en amont en ayant les deux jambes de la boucle solidarisées à la gaine de protection (120). Cette boucle (30) et sa solidarisation à la gaine intermédiaire (120) sont bien visibles sur les figures agrandies 3, 4 et 5. A l'intérieur de la boucle (30), passe un fil de traction (40) ayant une fonction de palan dont une extrémité (40a) est fixée au boîtier ou coque ici gauche (12) de la poignée (10) pour être fixe et l'autre extrémité (40b) est solidaire d'une roulette de manœuvre (50) montée à rotation sur le boîtier ou coque (12) de la poignée.

Selon une autre variante de réalisation particulière, la poignée (10) présente une partie avant (12a) ici sur la coque (12) en direction du stent (2) et une partie arrière (12b), pourvue d'une canule (112), servant à l'introduction d'un fil-guide, non représenté ici, passant dans le canal central (111). En position initiale, représentée aux figures 1 à 8, le système de palan (20) se trouve vers l'avant (12a) de la poignée (10) de manière que lors du retrait, par traction, la distance de retrait (D) soit d'une longueur sensiblement proche de la longueur de la poignée, cette longueur étant elle-même calculée en correspondance avec la longueur du stent (2).

Selon une autre variante de réalisation particulière, la roulette (50) comprend un arbre latéral (60) sur lequel vient s'enrouler le fil de traction (40).

Selon encore une variante de réalisation particulière, la roulette (50) avec son arbre latéral (60) se trouve dans une position intermédiaire entre l'avant (12a) et l'arrière (12b) de la poignée (10), la poignée (10) étant d'une dimension permettant d'être tenue confortablement dans la main d'un opérateur. L'homme de l'art comprend aisément que l'opérateur peut tenir fermement la poignée (10) tout en pouvant manœuvrer la roulette (50).

Selon encore une autre variante de réalisation particulière, la roulette (50) est montée sur un axe de rotation (51) positionné pour obtenir que le bord circonférentiel externe de la roulette (50) fasse saillie à l'extérieur de la poignée (10), en passant à travers une ouverture (70) prévue à cet effet dans le boîtier.

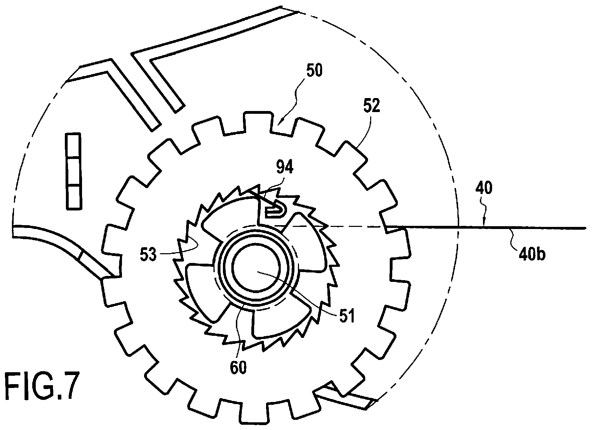

Selon une autre variante de réalisation particulière, le bord circonférentiel externe de la roulette (50) est cranté en présentant des crans (52), afin de faciliter l'opération de rotation exercée par un doigt de l'opérateur qui peut être le pouce lorsque la roulette (50) se trouve positionnée sur la partie supérieure de la poignée (10), comme représenté. Selon une variante, il peut être aussi également prévu au niveau de la roulette (50) la présence d'une lame (94) anti-retour de la roulette venant coopérer avec des dents (53) prévues dans un logement axial de la roulette (50), en empêchant sa rotation intempestive lors de l'insertion du cathéter (100), comme bien visible à la figure 7.

Selon encore une autre variante de réalisation particulière, il est prévu à l'intérieur et à l'arrière (12b) de la poignée (10), un axe support (82) de renvoi du fil de traction (40) provenant de la partie avant (12a) de la poignée (10) en passant dans la boucle (30), ce qui participe également à la réduction significative de la force de traction qu'il faut exercer sur la gaine intermédiaire (120) de protection pour la tracter à l'intérieur de la poignée (10) et libérer le stent (2).

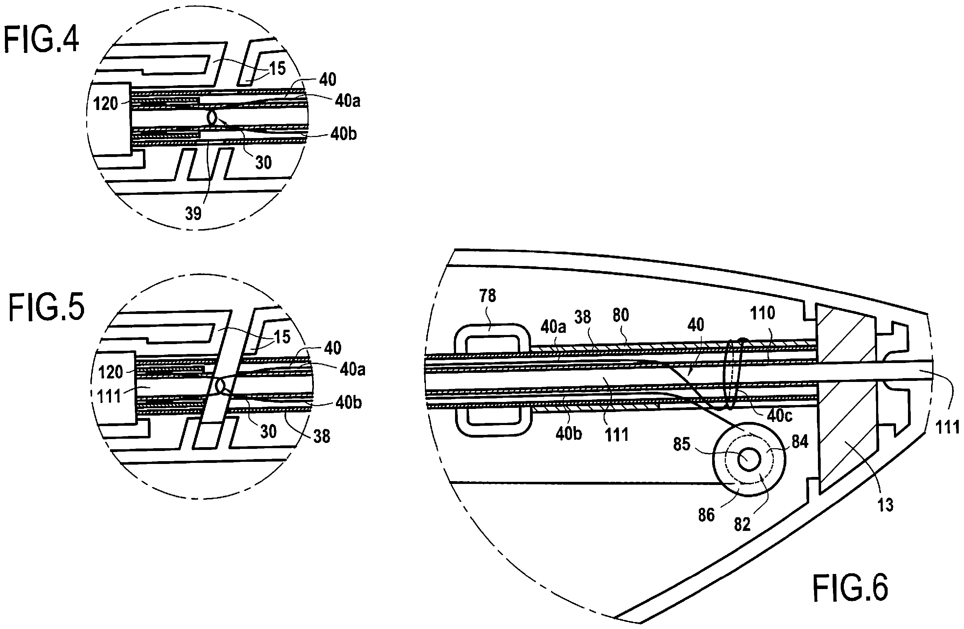

Selon une autre variante de réalisation particulière, l'axe support (82) de renvoi disposé à l'arrière de la poignée comprend une entretoise (84) solidaire de l'axe de support (82). La poignée peut aussi présenter à l'arrière un élément de renfort transversal (13) comprenant un orifice traversant définissant une continuité au canal central (111), voir figure 6.

Selon encore une variante de réalisation particulière, la poignée (10) présente à l'avant (12a) un élément de renfort (18) creux dans lequel passe le cathéter (100) évitant au dispositif cathéter (100) comprenant le stent (2) de se plicaturer. L'élément de renfort (18) présente selon une variante de réalisation représentée notamment à la figure 3, de manière très agrandie, une partie avant tronconique disposée à l'extérieur de la poignée et une partie arrière en forme de T inversé dont la base du T vient se loger dans un logement transversal où il est maintenu fermement en position fermée de la poignée.

Selon une autre variante de réalisation particulière, il est prévu un élément (90) de blocage provisoire d'un déplacement intempestif du système de palan (20).

Selon une variante de réalisation particulière, l'élément de blocage (90), tel qu'une goupille présentant un orifice (91) de passage d'un doigt pour la tirer, peut être prévu au niveau de la boucle (30) pour être par exemple introduit sous la boucle (30), et présenter par exemple une patte cylindrique (92) ou tronconique, afin de la soulever, et l'écraser pour la bloquer en position jusqu'au moment de l'opération de la poignée (10). L'élément de blocage (90) présente ici avantageusement une deuxième patte (93) venant s'insérer entre deux dents (52) de la roulette (50), ce qui l'empêche aussi de tourner intempestivement.

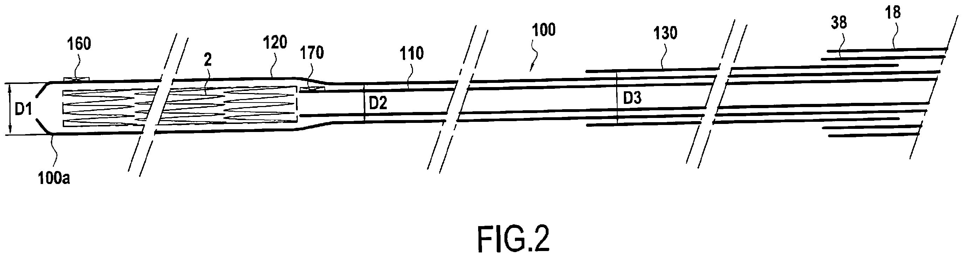

Selon un deuxième aspect l'invention concerne aussi un Système de délivrance d'un stent (2) positionné sur un cathéter (100), mieux représenté aux figures 1 et 2, caractérisé en ce qu'il comprend une poignée (10) telle que définie précédemment.

Selon une variante de réalisation particulière, le Système est caractérisé en ce que le cathéter (100) comprend une extrémité distale (100a) comprenant le stent (2) à délivrer et une extrémité proximale (100b), et est conformé pour être introduit dans un canal du corps humain, en particulier un vaisseau sanguin, une veine, une artère et une application périphérique ; comprenant un élément tubulaire ou gaine dit intermédiaire (120) ayant une extrémité proximale et une extrémité distale ; un stent (2) disposé de manière contrainte à l'intérieur de l'extrémité distale de l'élément tubulaire intermédiaire (120) ; un élément tubulaire ou gaine dit interne (110), ayant une extrémité proximale et une extrémité distale, disposé à l'intérieur de l'élément tubulaire dit intermédiaire (120) et ayant une longueur suffisante pour que son extrémité distale vienne se positionner à l'arrière du stent et le bloquer en position, pour empêcher son retrait, comme visible à la figure 2 ; l'extrémité proximale de l'élément tubulaire interne (110) est solidarisée à la poignée (10) pour avoir une position fixe.

Selon une autre variante de réalisation particulière de l'invention, il est prévu un élément tubulaire ou gaine dit externe (130), ayant une extrémité proximale et une extrémité distale, qui est positionné à l'extérieur de l'élément tubulaire intermédiaire (120), d'une dimension plus courte que l'élément tubulaire intermédiaire pour avoir son extrémité distale disposée à une distance prédéterminée en amont du stent, comme représenté, l'extrémité proximale de l'élément tubulaire externe (130) est solidarisée à la poignée (10) pour avoir aussi une position fixe.

Selon une autre variante de réalisation de l'invention, l'extrémité distale de l'élément tubulaire intermédiaire (120) présente un diamètre D1 plus grand sur au moins toute la longueur où il reçoit le stent (2) pour faciliter son insertion, puis présente une pente de jonction pour aboutir à un diamètre D2 plus petit du reste de l'élément tubulaire intermédiaire, comme montré à la figure 2. Ce diamètre D1 est prévu correspondre sensiblement au diamètre D3 de l'élément tubulaire externe (130) de manière à venir se positionner contre l'extrémité distale de l'élément tubulaire externe qui est fixe et qui constitue une butée d'arrêt du retrait de l'élément tubulaire intermédiaire.

Selon une autre variante, il est prévu à l'intérieur de la poignée un élément tubulaire (38) qui peut être prévu transparent qui sert de guide de déplacement des éléments mobiles comme la gaine intermédiaire (120) et le fil de traction (40).

L'homme de l'art comprend aisément que l'élément tubulaire intermédiaire (120) est prévu pour coulisser longitudinalement entre l'élément tubulaire interne (110) et l'élément tubulaire externe (130) qui restent de position fixe relativement à la poignée (10) en étant solidarisés à celle-ci ; le stent (2) restant en place car la position de l'extrémité distale de l'élément tubulaire interne (110) reste fixe et empêche tout mouvement de retrait du stent. L'invention permet donc de délivrer le stent (2) à une position précise.

L'homme de l'art comprend aussi que l'élément tubulaire dit externe (130) permet de diminuer les frottements avec le canal du corps humain dans lequel le cathéter (100) est introduit.

L'intérêt de la poignée selon l'invention, contrairement au système « point fixe» antérieur qui nécessite l'aide d'un assistant, consiste dans le fait que le chirurgien peut l'utiliser tout seul.

En effet, avec une de ses mains, le chirurgien ou l'opérateur peut corriger au niveau de l'introducteur, non représenté ici, tout mouvement non désiré de l'élément tubulaire dit intermédiaire (120) ou gaine de protection du stent (2) dans le canal tel qu'un vaisseau sanguin du patient qui pourrait engendrer un déploiement de l'implant ou stent (2) en dehors du site de la sténose. Et avec l'autre main, il peut, une fois l'élément de blocage (90) retiré, formant une goupille de sécurité, agir en rotation sur la roulette (50) afin de libérer le stent (2).

Il peut être avantageusement prévu, de manière classique, des marqueurs de position, par exemple un marqueur distal (160) à l'extrémité avant 100a du cathéter, ici sur l'élément tubulaire intermédiaire (120) ; un marqueur distal (170) à l'extrêmité avant de l'élément tubulaire interne (110) venant à l'arrière du stent (2) pour empêcher tout retrait de ce dernier. Ainsi, la position du stent (2) est parfaitement repérée pour le praticien.

La procédure de mise en place de l'implant ou stent (2) dans le canal tel qu'un vaisseau sanguin du patient est décrite ci-après :
- un introducteur est posé dans l'artère du patient : approche brachiale ou fémorale ;
- un fil- guide est inséré dans l'introducteur et franchit puis dépasse le site où l'implant ou stent (2) qui peut être avantageusement à mémoire de forme est prévu d'être implanté.

Le cathéter (100) est ensuite coulissé par son canal interne (111) sur le fil-guide et inséré dans l'introducteur pour s'avancer jusqu'au site de délivrance du stent (2), la position d'avancement du stent (2) étant repérée grâce aux marqueurs (160) et (170). Pendant cette mise en place, le cathéter (100) est avantageusement flushé par un mélange eau/liquide de contraste afin d'évacuer toute trace d'air de l'intérieur du cathéter comprenant les éléments ou gaines respectivement interne (110), intermédiaire (120) supportant l'implant ou stent (2) et réalisant sa protection du stent, et externe (130) facilitant l'introduction du cathéter.

On comprend ainsi que l'extrémité distale (100a) du cathéter (100) est engagée sur le fil-guide à travers l'introducteur afin de rallier le futur site d'implantation.

L'opérateur vérifie alors l'emplacement de la sténose en injectant un mélange de produit de contraste dans l'arborescence artérielle visible à la scopie afin de positionner l'implant ou stent (2) à l'endroit de la sténose.

Cet implant ou stent (2) est destiné à être implanté dans les artères périphériques, à l'emplacement où se trouvent des sténoses (diminution du diamètre de l'artère).

Les emplacements privilégiés sont les extrémités des membres du corps humain, en particulier les jambes, qui peuvent souffrir d'une mauvaise irrigation, derrière les genoux, etc..
L'implant ou stent (2) doit être souple pour absorber toute flexion, torsion, etc... créée par une mobilisation naturelle des membres.

L'intérêt de l'utilisation d'un matériau à mémoire de forme, par exemple en nitinol, pour fabriquer l'implant ou stent, permet à l'implant de reprendre sa forme initiale après avoir été positionné dans une artère superficielle ayant subi un choc (coin de table, etc ...) contrairement aux implants par exemple en inox ou CRCO, non à mémoire de forme, qui resteraient déformés.

Pour exemple, un stent de diamètre 7mm sera implanté dans la sténose d'une artère dont les diamètres en amont et aval de la sténose sont d'environ 6mm.

L'implant sera donc parfaitement apposé à la paroi et ainsi maintenu dans le flux artériel par la contrepression de celle ci.

### Définition des différents éléments de la poignée selon l'invention

La poignée (10) est formée de deux parties principales complémentaires ou coques droite (11) visible à la figure 1 et gauche (12) visible sur les figures 3 à 8.

La forme des coques gauche et droite a été conçue dans le but que le chirurgien puisse manipuler la poignée avec aisance et facilité.

La taille de la poignée est relativement faible afin de ne pas avoir un dispositif trop massif et un emballage trop encombrant tout en garantissant un recul de l'élément ou gaine intermédiaire suffisant quel que soit le stent de la gamme.

Le renfort (18) est situé à l'extrémité avant de la poignée (10), son rôle est d'éviter aux gaines de se plicaturer suite à des manipulations parfois brutales des opérateurs qui cherchent à positionner correctement le stent au niveau de la sténose.

Le renfort est souple tout en étant suffisamment résistant pour jouer son rôle de protection.

### La roulette (50):

Le fil (40) accroché à la roulette (50) a la possibilité de s'enrouler autour, en particulier ici autour de son arbre latéral (60) lorsque le chirurgien décide de libérer le stent (2) en la faisant tourner. La roulette (50) tend le fil (40) qui passe par la boucle (30) de l'élément tubulaire ou gaine intermédiaire (120). Le fil (40) étant fixé à son autre extrémité (40a), le système décrit parfaitement un palan. Cette structure divise par deux la force de traction nécessaire au retrait de l'élément tubulaire ou gaine intermédiaire (120).
Les diamètres de la roulette (50) et de l'arbre latéral (60) ont été optimisés en vue de faciliter la libération du stent (2) : diamètre d'enroulement du fil (40) et diamètre hors tout de la roulette (50), où se trouvent les crans (52).
Les crans (52) ont été dessinés afin de ne pas avoir une forme trop agressive pour les gants de l'opérateur tout en lui procurant une accroche suffisante pour ne pas riper. Un système de cliquet anti-retour (94), par exemple une lame flexible comme une lame de pile empêche tout mouvement inverse de la roulette (50).
La cale 78 : Son rôle est minime mais permet lors de l'assemblage le positionnement aisé des différents composants internes à la poignée. L'embase (80) est située dans le prolongement de l'élément tubulaire interne 110 aussi dénommé « push tubing » ou « stop tubing » qui est lui même collé à l'intérieur de l'embase (80).
L'embase (80) et l'élément tubulaire interne (110) permettent le passage du fil- guide dans le canal central (111) sur toute la longueur du système de délivrance comprenant le cathéter (100).
Le fil (40) du palan (20) est aussi fixé à l'embase (80) à l'intérieur de celle ci.

Le tube transparent (38) s'étend à l'intérieur de la poignée sur presque toute sa longueur. Il présente une échancrure (39) pour le passage de la patte de l'organe de blocage. Le fil (40) part de l'intérieur de l'embase (80) où il est fixé avec aussi un nœud 42 sur la coque (12) en direction de la boucle (30) de la gaine grise intermédiaire puis revient à l'arrière vers le support de renvoi (82) afin de le rediriger vers la roulette (50) et son palier d'enroulement (60).
Le tube transparent (78) permet de maintenir droit dans la poignée, le guide, l'élément tubulaire interne (110), et l'élément intermédiaire (120) lors du retrait de cette dernière, il est à noter que l'élément tubulaire externe (130) est fixé à la partie avant de la poignée (10). Ainsi, Le tube transparent (78) est collé à la coque gauche (12) afin qu'il reste bien raide lors de la mise sous tension.
Dans l'espace prévu entre l'extérieur de l'élément tubulaire interne (110) ou « push tubing » et l'intérieur de la gaine transparente (78), le palan (20) exerce sa fonction.
La boucle (30) peut être constituée par un fil, ayant deux jambes collées sur l'élément tubulaire intermédiaire (120). Le fil (40) sert aussi à mobiliser l'élément tubulaire intermédiaire (120) en le tirant en passant à travers sa boucle (30), via le système de palan (20).
Un support de renvoi ou goupille métallique (82) collé(e) dans son emplacement de la coque gauche (12) qui sert d'axe solide afin de pouvoir renvoyer le fil de l'embase vers la roulette. La goupille métallique (82) est aussi maintenue par la coque droite (11) lors de la fermeture de la poignée (10).
Une entretoise (84) collée autour de la goupille métallique (82) sert à augmenter le rayon de courbure de celle-ci afin que le renvoi soit efficace et progressif.
- Un élément (94) anti-retour, par exemple une lame de pile, positionné(e) sous la partie axiale de la roulette (50), sert à empêcher tout retour de la roulette lors de la mise sous tension du fil (40).

### Conclusion

Le système de palan (20) selon l'invention permet de diviser par deux la force exercée par l'opérateur sur la roulette (50) afin de faire reculer ou tracter l'élément ou gaine intermédiaire (120). La boucle (30) collée à la base de l'élément ou gaine intermédiaire (120) fait office de « poulie » dans laquelle le fil (40) de retrait, relié d'une part au boîtier ou ici à l'embase solidaire du boîtier qui constitue un point fixe, et d'autre part à la roulette (50) qui constitue un point mobile, coulisse.

## Revendications

1. Poignée (10) pour délivrance d'un stent (2) par mouvement de retrait d'un élément tubulaire ou gaine (120) de protection du stent (2), comprenant un système de palan (20) servant à la traction de la gaine de protection (120) sur une distance de retrait (D) prédéterminée afin de découvrir le stent et permettre sa délivrance et sa dilatation, **caractérisée en ce que** le système de palan (20) comprend une boucle (30) solidaire de la gaine de protection (120) en amont dans laquelle passe un fil de traction (40) ayant une fonction de palan dont une extrémité (40a) est fixée au boîtier (11) de la poignée (10) pour être fixe et l'autre extrémité (40b) est solidaire d'une roulette de manœuvre (50) montée à rotation sur le boîtier (11) de la poignée.

2. Poignée selon la revendication 1, **caractérisée en ce que** la poignée (10) présente une partie avant (12a) en direction du stent (2) et une partie arrière (12b) servant à l'introduction d'un cathéter (100) sur lequel est monté le stent (2) ; en position initiale, le système de palan (20) se trouve vers l'avant (12a) de la poignée de manière que lors du retrait, par traction, la distance de retrait (D) soit d'une longueur sensiblement proche de la longueur de la poignée, cette longueur étant elle-même calculée en correspondance avec la longueur du stent (2).

3. Poignée selon l'une des revendications 1 ou 2, **caractérisée en ce que** la roulette (50) précitée comprend un arbre latéral (60) sur lequel vient s'enrouler le fil de traction (40).

4. Poignée selon la revendication 3, **caractérisée en ce que** la roulette (50) avec son arbre latéral (60) se trouve dans une position intermédiaire entre l'avant (12a) et l'arrière (12b) de la poignée (10), la partie arrière (12b) de la poignée (10) étant d'une dimension permettant d'être tenue confortablement dans la main d'un opérateur.

5. Poignée selon l'une des revendications 1 à 4, **caractérisée en ce que** la roulette (50) est montée sur un axe de rotation (51) positionné pour obtenir que le bord circonférentiel externe de la roulette (50) fasse saillie à l'extérieur de la poignée (10), en passant à travers une ouverture (70) prévue à cet effet dans le boîtier.

6. Poignée selon la revendication 5, **caractérisée en ce que** le bord extérieur circonférentiel de la roulette (50) est cranté en présentant des crans (52), afin de faciliter l'opération de rotation exercée par un doigt de l'opérateur qui peut être le pouce.

7. Poignée selon l'une des revendication 1 à 6, **caractérisée en ce qu'**il est prévu à l'intérieur et à l'arrière (12b) de la poignée (10), un axe support (82) de renvoi du fil de traction (40) provenant de la partie avant (12a) de la poignée (10) en passant dans la boucle (30), ce qui participe également à l'abaissement de la force de traction qu'il faut exercer sur la gaine de protection (120) pour la tracter à l'intérieur de la poignée (10).

8. Poignée selon la revendication 7, **caractérisée en ce que** l'axe support (82) de renvoi disposé à l'arrière de la poignée comprend une entretoise (84) solidaire de l'axe de support (82).

9. Poignée selon l'une des revendication 1 à 8, **caractérisée en ce que** la poignée (10) présente à l'avant (10a) un élément de renfort (18) creux dans lequel passe le cathéter (100) évitant au dispositif cathéter (100) comprenant le stent (2) de se plicaturer.

10. Poignée selon l'une des revendications 1 à 9, **caractérisée en ce qu'**il est prévu un élément (90) de blocage provisoire d'un déplacement du système de palan (20).

11. Poignée selon la revendication 10, **caractérisée en ce que** l'élément de blocage (90) peut être prévu au niveau de la boucle (30) pour être par exemple introduit sous la boucle (30), et présenter par exemple une patte cylindrique (92) ou tronconique, afin de la soulever, et l'écraser pour la bloquer en position jusqu'au moment de l'opération de la poignée (10) et avantageusement aussi au niveau de la roulette (50) en empêchant sa rotation.

12. Système (1) de délivrance d'un stent (2) positionné sur un cathéter (100), **caractérisé en ce qu'**il comprend une poignée (10) telle que définie à l'une quelconque des revendications précédentes.

13. Système selon la revendication 12, **caractérisé en ce que** le cathéter (100) comprend une extrémité distale (100a) comprenant le stent (2) à délivrer et une extrémité proximale (100b), et est conformé pour être introduit dans un canal du corps humain, en particulier un vaisseau sanguin, une veine, une artère périphérique ; ledit cathéter (100) comprenant un élément tubulaire ou gaine dit intermédiaire (120) ayant une extrémité proximale et une extrémité distale ; un stent (2) disposé de manière contrainte à l'intérieur de l'extrémité distale de l'élément tubulaire intermédiaire (120) ; un élément tubulaire ou gaine dit interne (110), ayant une extrémité proximale et une extrémité distale, disposé à l'intérieur de l'élément tubulaire dit intermédiaire (120) et ayant une longueur suffisante pour que son extrémité distale vienne se positionner à l'arrière du stent (2) et le bloquer en position, pour empêcher son retrait ; l'extrémité proximale de l'élément tubulaire interne (110) est solidarisée à la poignée pour avoir une position fixe.

14. Système selon la revendication 12 ou 13, **caractérisé en ce que** le cathéter (100) comprend un élément tubulaire dit externe (130) prévu pour diminuer les frottements avec le canal du corps humain, qui est positionné à l'extérieur de l'élément tubulaire intermédiaire, (120) d'une dimension plus courte que l'élément tubulaire intermédiaire (120) pour avoir son extrémité distale disposée à une distance prédéterminée en amont du stent, l'extrémité proximale de l'élément tubulaire externe (130) est solidarisée à la poignée pour avoir aussi une position fixe.

15. Système selon la revendication 13 ou 14, **caractérisé en ce que**, l'extrémité distale de l'élément tubulaire intermédiaire (120) présente un diamètre D1 plus grand sur toute la longueur où il reçoit le stent pour faciliter son insertion, puis présente une pente de jonction pour aboutir à un diamètre D2 plus petit sur le reste de l'élément tubulaire intermédiaire (120) ; ce diamètre D1 est avantageusement prévu pour correspondre sensiblement au diamètre D3 de l'élément tubulaire externe (130) de manière à venir se positionner contre l'extrémité distale de l'élément tubulaire externe (130) qui est fixe et qui constitue une butée d'arrêt du retrait de l'élément tubulaire intermédiaire.

## Patentansprüche

1. Griff (10) zur Freisetzung eines Stents (2) durch Entnahmebewegung eines röhrenförmigen oder umhüllenden Schutzelements (120) des Stents (2), umfassend ein Hebesystem (20), das dem Zug der Schutzhülle (120) über eine Entnahmestrecke (D) dient, die vorbestimmt ist, um den Stent aufzudecken und seine Freisetzung und seine Dilatation zuzulassen, **dadurch gekennzeichnet, dass** das Hebesystem (20) eine Schleife (30) umfasst, die einstückig mit der Schutzhülle (120) vorgelagert ist, in der ein Zugdraht (40) durchläuft, der eine Hebefunktion aufweist, wobei ein Ende (40a) an dem Gehäuse (11) des Griffes (10) befestigt ist, um fest zu sein, und das andere Ende (40b) einstückig mit einer Betätigungsrolle (50) ist, die drehbar auf dem Gehäuse (11) des Griffes montiert ist.

2. Griff nach Anspruch 1, **dadurch gekennzeichnet, dass** der Griff (10) einen vorderen Teil (12a) in Richtung des Stents (2) und einen hinteren Teil (12b) aufweist, welcher der Einführung eines Katheters (100) dient, auf dem der Stent (2) montiert ist, wobei sich das Hebesystem (20) in Ausgangsposition zu der Vorderseite (12a) des Griffes hin befindet, sodass während der Entnahme durch Zug die Entnahmestrecke (D) eine Länge aufweist, die im Wesentlichen nahe der Länge des Griffes ist, wobei diese Länge selbst in Übereinstimmung mit der Länge des Stents (2) berechnet ist.

3. Griff nach einem der Ansprüche 1 oder 2, **dadurch gekennzeichnet, dass** die vorstehend genannte Rolle (50) eine Seitenwelle (60) umfasst, auf der sich der Zugdraht (40) einrollt.

4. Griff nach Anspruch 3, **dadurch gekennzeichnet, dass** sich die Rolle (50) mit ihrer Seitenwelle (60) in einer Zwischenposition zwischen der Vorderseite (12a) und der Rückseite (12b) des Griffes (10) befindet, wobei der hintere Teil (12b) des Griffes (10) eine Abmessung aufweist, die ein komfortables Halten in der Hand eines Bedieners zulässt.

5. Griff nach einem der Ansprüche 1 bis 4, **dadurch gekennzeichnet, dass** die Rolle (50) auf einer Drehachse (51) montiert ist, die positioniert ist, um zu erreichen, dass der äußere Umfangsrand der Rolle (50) aus dem Äußeren des Griffes (10) herausragt, während er eine Öffnung (70) durchläuft, die zu diesem Zweck in dem Gehäuse vorgesehen ist.

6. Griff nach Anspruch 5, **dadurch gekennzeichnet, dass** der äußere Umfangsrand der Rolle (50) gewellt ist, indem er Rastungen (52) aufweist, um den Betrieb der Drehung zu erleichtern, der durch einen Finger des Betreibers ausgeübt wird, welcher der Daumen sein kann.

7. Griff nach einem der Ansprüche 1 bis 6, **dadurch gekennzeichnet, dass** im Inneren und an der Rückseite (12b) des Griffes (10) eine Stützachse (82) zur Rückführung des Zugdrahtes (40) vorgesehen ist, die von dem vorderen Teil (12a) des Griffes (10) ausgeht und die Schleife (30) durchläuft, was auch zum Reduzieren der Zugkraft, die auf die Schutzhülle (120) ausgeübt werden muss, um sie in das Innere des Griffes (10) zu ziehen, beiträgt.

8. Griff nach Anspruch 7, **dadurch gekennzeichnet, dass** die Stützachse (82) zur Rückführung, die hinter dem Griff angeordnet ist, einen Abstandshalter (84) umfasst, der einstückig mit der Stützachse (82) ist.

9. Griff nach einem der Ansprüche 1 bis 8, **dadurch gekennzeichnet, dass** der Griff (10) auf der Vorderseite (10a) ein hohles Verstärkungselement (18) aufweist, in dem der Katheter (100) verläuft, wodurch verhindert wird, dass die Kathetervorrichtung (100), die den Stent (2) umfasst, sich abbindet.

10. Griff nach einem der Ansprüche 1 bis 9, **dadurch gekennzeichnet, dass** ein provisorisches Blockierungselement (90) zur Verschiebung des Hebesystems (20) vorgesehen ist.

11. Griff nach Anspruch 10, **dadurch gekennzeichnet, dass** das Blockierungselement (90) auf Höhe der Schleife (30) vorgesehen sein kann, um zum Beispiel unter die Schleife (30) eingeführt zu werden, und zum Beispiel ein zylindrisches (92) oder kegelstumpfförmiges Muster aufweisen kann, um sie anzuheben, und sie zu zerdrücken, um sie genau in dem Moment der Betätigung des Griffes (10) in Position zu blockieren, und vorteilhafterweise auch auf Höhe der Rolle (50), indem ihre Drehung verhindert wird.

12. System (1) zur Freisetzung eines Stents (2), der auf einem Katheter (100) positioniert ist, **dadurch gekennzeichnet, dass** es einen Griff (10) wie in einem der vorhergehenden Ansprüche definiert umfasst.

13. System nach Anspruch 12, **dadurch gekennzeichnet, dass** der Katheter (100) ein distales Ende (100a), das den freizusetzenden Stent (2) umfasst, und ein proximales Ende (100b) umfasst und ausgebildet ist, um in einen menschlichen Körperkanal eingeführt zu werden, insbesondere ein Blutgefäß, eine Vene, eine periphere Arterie, wobei der Katheter (100) ein röhrenförmiges oder umhüllendes Element umfasst, das als Zwischenelement (120) bezeichnet wird, das ein proximales Ende und ein distales Ende aufweist, wobei ein Stent (2) auf einschränkende Weise im Inneren des distalen Endes des röhrenförmigen Zwischenelements (120) angeordnet ist, wobei ein röhrenförmiges oder umhüllendes Element, das als Innenelement (110) bezeichnet wird, das ein proximales Ende und ein distales Ende aufweist, im Inneren des röhrenförmigen Elements angeordnet ist, das als Zwischenelement (120) bezeichnet wird und eine Länge aufweist, die ausreichend ist, damit sich sein distales Ende hinter dem Stent (2) positioniert und seine Position blockiert, um seine Entnahme zu verhindern, wobei das proximale Ende des röhrenförmigen Innenelements (110) fest mit dem Griff verbunden ist, um eine feste Position aufzuweisen.

14. System nach Anspruch 12 oder 13, **dadurch gekennzeichnet, dass** der Katheter (100) ein röhrenförmiges Element umfasst, das als Außenelement (130) bezeichnet wird, das vorgesehen ist, um die Reibungen mit dem menschlichen Körperkanal zu reduzieren, und das außerhalb des röhrenförmigen Zwischenelements (120) positioniert ist, mit einer kürzeren Abmessung als das röhrenförmige Zwischenelement (120), sodass sein distales Ende in einem zuvor festgelegten Abstand vor dem Stent angeordnet ist, wobei das proximale Ende des röhrenförmigen Außenelements (130) fest mit dem Griff verbunden ist, um ebenfalls eine feste Position aufzuweisen.

15. System nach Anspruch 13 oder 14, **dadurch gekennzeichnet, dass** das distale Ende des röhrenförmigen Zwischenelements (120) einen Durchmesser D1 aufweist, der über die gesamte Länge, an der er den Stent aufnimmt, größer ist, um seine Einführung zu erleichtern, dann eine Verbindungssteigung aufweist, um auf dem Rest des röhrenförmigen Zwischenelements (120) einen kleineren Durchmesser D2 zu erreichen, wobei dieser Durchmesser D1 vorteilhafterweise vorgesehen ist, um im Wesentlichen dem Durchmesser D3 des röhrenförmigen Außenelements (130) derart zu entsprechen, um sich gegen das distale Ende des röhrenförmigen Außenelements (130) zu positionieren, das fest ist und das einen Entnahmeanschlag des röhrenförmigen Zwischenelements darstellt.

## Claims

1. A handle (10) for delivering a stent (2) by retracting a tubular element or sheath (120) for protecting the stent (2), including a tackle system (20) used to pull the protective sheath (120) over a predetermined retraction distance (D) to uncover the stent and enable the stent to be delivered and expanded, **characterized in that** the tackle system (20) includes a loop (30) that is rigidly connected on the upstream side to the protective sheath (120), through which passes a traction wire (40) performing a tackle function, one end (40a) of which is fastened to the housing (11) of the handle (10), and is therefore static, and the other end (40b) of which is rigidly connected to a control wheel (50) that is mounted in rotation on the housing (11) of the handle.

2. The handle as claimed in claim 1, **characterized in that** the handle (10) has a front portion (12a) on the side of the stent (2) and a rear portion (12b) used to insert a catheter (100) on which the stent (2) is mounted; in a starting position, the tackle system (20) is positioned towards the front (12a) of the handle so that, when retracted by traction, the retraction distance (D) is substantially close to the length of the handle, this length being calculated in correspondence with the length of the stent (2).

3. The handle as claimed in one of claims 1 or 2, **characterized in that** the wheel (50) has a lateral shaft (60) onto which the traction wire (40) is wound.

4. The handle as claimed in claim 3, **characterized in that** the wheel (50) and the related lateral shaft (60) are in an intermediate position between the front (12a) and rear (12b) of the handle (10), the size of the rear portion (12b) of the handle (10) being such as to enable the handle to be held comfortably in an operator's hand.

5. The handle as claimed in one of claims 1 to 4, **characterized in that** the wheel (50) is mounted on a rotary shaft (51) positioned such as to ensure that the external circumferential edge of the wheel (50) projects outside the handle (10), passing through an opening (70) provided for this purpose in the housing.

6. The handle as claimed in claim 5, **characterized in that** the external circumferential edge of the wheel (50) is notched and has notches (52) to facilitate the rotational movement imparted by the operator's finger or thumb.

7. The handle as claimed in one of claims 1 to 6, **characterized in that** a support and change-of-direction shaft (82) for the traction wire (40) coming from the front portion (12a) of the handle (10) through the loop (30) is provided inside the rear portion (12b) of the handle (10), which also helps to lower the tractive force that needs to be exerted on the protective sheath (120) to pull said sheath inside the handle (10).

8. The handle as claimed in claim 7, **characterized in that** the support and change-of-direction shaft (82) arranged at the rear of the handle includes a spacer (84) that is rigidly connected to the support shaft (82).

9. The handle as claimed in one of claims 1 to 8, **characterized in that** the handle (10) has a hollow reinforcement element (18) at the front (10a) into which the catheter (100) is inserted, thereby preventing the catheter device (100) including the stent (2) from kinking.

10. The handle as claimed in one of claims 1 to 9, **characterized in that** an element (90) for temporarily blocking movement of the tackle system (20) is provided.

11. The handle as claimed in claim 10, **characterized in that** the blocking element (90) can be provided at the loop (30), for example by insertion beneath the loop (30), and can for example have a cylindrical or tapered lug (92) designed to raise and compress the loop in order to block the loop in position until the handle (10) is used, and advantageously also at the wheel (50) to prevent rotation thereof.

12. A system (1) for delivering a stent (2) positioned on a catheter (100), **characterized in that** it includes a handle (10) as defined in any one of the preceding claims.

13. The system as claimed in claim 12, **characterized in that** the catheter (100) has a distal end (100a) including the stent (2) to be delivered and a proximal end (100b), and is shaped to be inserted into a channel of the human body, in particular a blood vessel, vein or peripheral artery; said catheter (100) including an intermediate tubular element or sheath (120) with a proximal end and a distal end; a stent (2) arranged in a constrained manner inside the distal end of the intermediate tubular element (120); an inner tubular element or sheath (110) with a proximal end and a distal end that is arranged inside the intermediate tubular element (120) and that is long enough that the distal end thereof is positioned at the rear of the stent (2) and blocks the stent in position to prevent the stent from being retracted; the proximal end of the inner tubular element (110) is rigidly connected to the handle and is therefore static.

14. The system as claimed in claim 12 or 13, **characterized in that** the catheter (100) includes an outer tubular element (130) that is provided to reduce friction with the channel of the human body, that is positioned outside the intermediate tubular element (120) and that is shorter than the intermediate tubular element (120) so that the distal end thereof is positioned at a predetermined distance upstream of the stent, and the proximal end of the outer tubular element (130) is rigidly connected to the handle, also in a static position.

15. The system as claimed in claim 13 or 14, **characterized in that** the distal end of the intermediate tubular element (120) has a diameter D1 that is greater over the entire length along which the stent is received, to facilitate the insertion thereof, then has an inclined joint resulting in a lesser diameter D2 over the remainder of the intermediate tubular element (120); this diameter D1 is advantageously substantially equal to the diameter D3 of the outer tubular element (130) such as to be positioned against the distal end of the outer tubular element (130), which is static and forms a stop preventing retraction of the intermediate tubular element.
